(19) Europäisches Patentamt
European Patent Office
Office européen des brevets

(11) Veröffentlichungsnummer: **0 305 913 B1**

(12) # EUROPÄISCHE PATENTSCHRIFT

(45) Veröffentlichungstag der Patentschrift: **22.05.91 Patentblatt 91/21**

(51) Int. Cl.[5]: **C07C 33/02**, C07C 29/60, C07C 29/10, C07C 43/15

(21) Anmeldenummer: **88113911.7**

(22) Anmeldetag: **26.08.88**

(54) **Verfahren zur Herstellung von Dien-1-olen, 9-Hydroxydodec-10-enyl-1-tert.-butylether und seine Verwendung als Zwischenprodukt zur Synthese von 8,10-Dodecadienol.**

(30) Priorität: **02.09.87 DE 3729225**

(43) Veröffentlichungstag der Anmeldung: **08.03.89 Patentblatt 89/10**

(45) Bekanntmachung des Hinweises auf die Patenterteilung: **22.05.91 Patentblatt 91/21**

(84) Benannte Vertragsstaaten: **AT BE CH DE ES FR GB IT LI NL SE**

(56) Entgegenhaltungen:
**EP-A- 0 003 708**
**CH-A- 578 490**
**CH-A- 622 760**
**DE-C- 2 123 434**
**GB-A- 2 098 609**

(56) Entgegenhaltungen:
**THE JOURNAL OF ORGANIC CHEMISTRY, Band 44, Nr. 21, 1979; JAMES H. BABLER et al.: "A Convenient Stereoselctive Route to the Sex Pheromone of the Red Bollworm Moth via an Allylic Sulfenate to Sulfoxide Rearrangement", Seiten 3723-3725**

(73) Patentinhaber: **BASF Aktiengesellschaft
Carl-Bosch-Strasse 38
W-6700 Ludwigshafen (DE)**

(72) Erfinder: **Mackenroth, Wolfgang, Dr.
Seebacher Strasse 37
W-6702 Bad Duerkheim (DE)**
Erfinder: **Hoelderich, Wolfgang, Dr.
Mannheimer Strasse 18 c
W-6710 Frankenthal (DE)**
Erfinder: **Becker, Rainer, Dr.
Im Haseneck 22
W-6702 Bad Duerkheim (DE)**
Erfinder: **Seufert, Walter, Dr.
Laerchenweg 19
W-6720 Speyer (DE)**

**EP 0 305 913 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von Dien-1-olen der allgemeinen Formel I

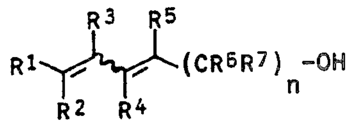

(I),

in der die Reste $R^1$ bis $R^7$ untereinander gleich oder verschieden sind und für Wasserstoff oder geradkettige oder verzweigte Alkylgruppen mit 1-12 Kohlenstoffatomen stehen und n = 1-14 bedeutet.

Insbesondere betrifft die Erfindung ein neues Verfahren zur Herstellung von 8,10-Dodecadienol (5) aus Octandiol, unter anderem über den 9-Hydroxydodec-10-enyl-1-tert.-butylether (4) nach dem nachstehenden Schema 1, wobei erfindungswesentlich die neue Verbindung (4) zur Verfügung gestellt wird.

Schema 1:

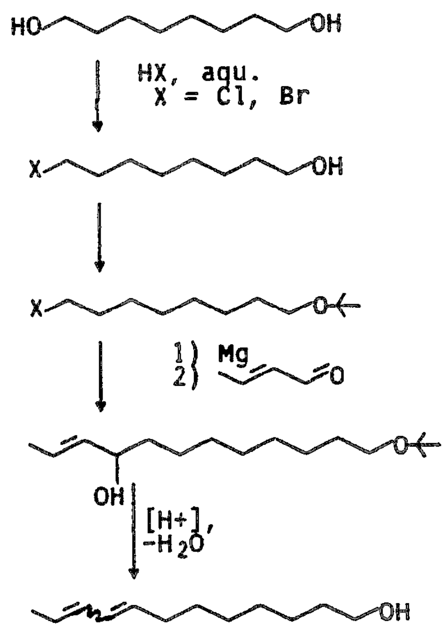


(1)
(2)
(3)
(4)
(5)

8,10-Dodecadienol (5) wirkt bei gewissen Insekten als Pheromon.

Der Pheromonwirkstoff wurde erstmals von ROELOFS et al beschrieben [DE 2 123 434, GB 1 299 691]. Die angegebenen Herstellverfahren bemühen entweder eine Vielzahl von meist aufwendigen Einzelstufen und komplizierten Reaktionen oder gehen von teuren Ausgangsstoffen aus ; sie sind somit für die Herstellung großer Mengen im technischen Maßstab wenig geeignet.

BABLER und INVERGO [J. Org. Chem. 44 (21), 3723 (1979)] beschreiben ein Verfahren zur Synthese von E-9,11-Dodecadien-1-ol bei dem als Zwischenprodukt ein tetrahydropyranylgeschützter Allylalkohol benutzt wird (Schema 2).

Schema 2:

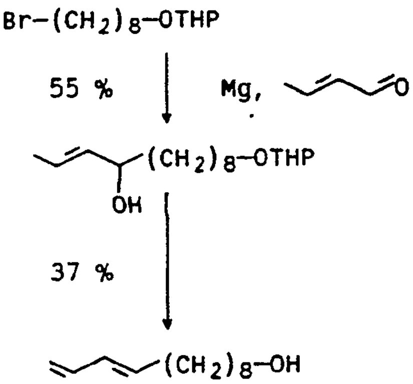


Dabei wird in einer Grignard-Reaktion durch die Schutzgruppe Tetrahydropyranyl (THP) geschütztes Bromoctanol in 55%iger Ausbeute zum Allylalkohol umgesetzt, der unter Verschiebung des Doppelbindungssystems zu E-9,11-Dodecadien-1-yl-tetrahydropyranylether dehydratisiert wird. In einem zusätzlichen Schritt wird die Schutzgruppe abgespalten. Nachteilig an diesem Verfahren ist einerseits die geringe Ausbeute bei der Herstellung des THP-geschützten Bromoctanols und andererseits die Notwendigkeit, die Schutzgruppe in einem zusätzlichen Verfahrensschritt abspalten zu müssen, wodurch die Gesamtausbeute des Verfahrens stark herabgesetzt wird.

Der Erfindung lag die Aufgabe zugrunde, ein vorteilhaftes Verfahren zur Herstellung von Dien-1-olen I zur Verfügung zu stellen. Insbesondere sollte ein einfacher Zugang zu 8,10-Dodecadienol (5) aufgefunden werden.

Demgemäß wurde ein Verfahren zur Herstellung von Dien-1-olen der allgemeinen Formel I

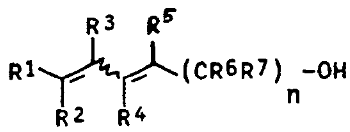
(I),

in der die Reste $R^1$ bis $R^7$ untereinander gleich oder verschieden sind und für Wasserstoff oder geradkettige oder verzweigte Alkylgruppen mit 1-12 Kohlenstoffatomen stehen und n = 1-14 bedeutet, gefunden, daß dadurch gekennzeichnet ist, daß man Hydroxyalkenyl-tert.-butylether der Formel II

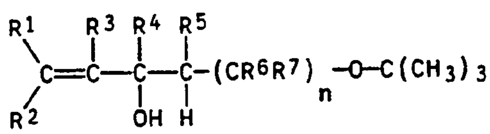
(II)

in Gegenwart von sauren Katalysatoren bei erhöhter Temperatur dehydratisiert und dabei im wesentlichen gleichzeitig die tert.-Butylschutzgruppe abspaltet.

Durch die Verwendung tert.-Butyl geschützter Allylalkohole, z.B. dem erfindungsgemäßen 9-Hydroxydoc-10-enyl-1-tert.-butylether (4) können die beiden Reaktionsschritte Dehydratisierung und Abspaltung der Schutzgruppe im wesentlichen gleichzeitig vorgenommen werden.

Die Verbindung (4) ist in an sich bekannter Weise aus Octandiol (1) durch Umsetzung mit der entsprechenden Halogenwasserstoffsäure zum 8-Halogenoctanol (2) [Rossi, Synthesis 359 (1981) ; HAPMAN et al J. Am. Chem. Soc. 100, 4878 (1979)] und nachfolgender Reaktion mit Isobuten zum tert.-Butylether (3) den man anschließend der Grignard-Reaktion mit Crotonaldehyd unterwirft, zugänglich.

Mit der erfindungsgemäß durch die Schutzgruppe t-Butyl geschützten Verbindung (4) gelingt die an sich analoge Grignard-Reaktion zum Allylalkohol mit erheblich höherer Ausbeute (÷85%). Die anschließende Dehydratisierung zum 8,10-Diensystem und Abspaltung der Schutzgruppe können in einem Schritt ohne Lösungsmittel in Gegenwart eines sauren Katalysators durchgeführt werden. Die Isolierung des geschützten Alkohols kann daher unterlassen werden.

Das erfindungsgemäße Verfahren insbesondere zur Synthese von 8,10-Dodecadienol (5) über den Allylalkohol (4) stellt somit einen neuen, vorteilhaften Weg dar.

In den Verbindungen der Formeln I bzw. II stehen die Reste $R^1$ bis $R^7$ für Wasserstoff oder Alkylgruppen mit 1-12, vorteilhaft 1-6, insbesondere 1 bis 4 Kohlenstoffatomen. Beispielsweise seien Methyl, Ethyl, Propyl, Isopropyl, n-Butyl und dessen Isomere genannt. Vorzugsweise stehen die Reste $R^2$ bis $R^7$ für Wasserstoff und $R^1$ für eine niedermolekulare Alkylgruppe, z.B. Methyl.

Vorteilhaft sind zwischen der Doppelbindung im Allylalkohol II und der geschützten OH-Gruppe mehrere Kohlenstoffatome vorhanden. So steht beispielsweise n für eine Zahl von 4 bis 14, besonders bevorzugt 6 bis 12.

Verbindungen, die nach dem erfindungsgemäßen Verfahren vorteilhaft hergestellt werden können, sind beispielsweise 8,10-Dodecadien-1-ol, 6,8-Decadien-1-ol, 4,6-Octadien-1-ol und 2,4-Hexadien-1-ol.

Für das erfindungsgemäße Verfahren ist die Wahl des sauren Katalysators nicht kritisch, so sind alle an sich bekannten Dehydratisierungsmittel einsetzbar wie Mineralsäuren, z.B. Schwefelsäure, Salzsäure, Phosphorsäure, Borsäure, organische Säuren wie Ameisensäure, Sulfonsäuren, z.B. p-Toluolsulfonsäure, Anhydride wie Phosphorpentoxid, Phthalsäureanhydrid, saure Salze wie Kaliumhydrogensulfat oder Kupfersulfat. Besonders bevorzugte Katalysatoren aus der Gruppe der Homogenkatalysatoren sind Schwefelsäure und p-Toluolsulfonsäure.

Es können vorteilhaft auch saure Heterogenkatalysatoren für die Dehydratisierung und Abspaltung der Schutzgruppe verwendet werden. Hier kommen insbesondere acide Zeolithe, Phosphate, Metalloxide der Elemente Si, Al, Ti, Zr, B, Fe, W, Mo, Nb und V oder Phosphor- oder Borsäure auf üblichen Trägermaterialien in Betracht. Beispielsweise seien folgende Heterogenkatalysatoren aufgeführt:

Zeolithe aus der Mordenit-Gruppe oder engporige Zeolithe vom Erionit- bzw. Chabasit-Type oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et. al. Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X und Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen ($SiO_2/Al_2O_3 \geqq 10$) gehören auch die sogenannten ZSM-Typen, Ferrierit, NU-1 und Silicalit® (ein Molekularsieb, sog. Silica Polymorph).

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Ver-

hältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amino, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metall werden Alkalimetall wie Li, Cs, K, Erdalkalimetall wie Mg, Ca, Sr, Metalle der 3., 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(No_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine wäßrige $Ni(CO_3)_2$-Lösung oder ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material – verformt oder unverformt – einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 25 gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450°C bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50°C bis 90°C, vorzugsweise 60°C bis 80°C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger $H_3PO_4$-Lösung getränkt, bei 110°C getrocknet und bei 500°C calciniert.

Weitere Katalysatoren für das erfindungsgemäße Verfahren sind Phosphate, insbesondere Aluminiumphosphate, Siliciumaluminiumphosphate, Siliciumeisenaluminiumphosphate, Ceriumphosphat, Zirkonphosphate, Boriumphosphat, Eisenphosphat oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt, die Zeolithstruktur besitzen.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und 4 473 663 beschrieben.

Beispielsweise wird das $AlPO_4$-5 (APO-5) synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte $AlPO_4$ wird getrocknet bei 100 bis 160°C und calciniert bie 450 bis 550°C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200°C unter autogenem Druck währen 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200°C unter autogenem Druck während 50 bis 200 h.

Für das erfindungsgemäße Verfahren eingesetzbaren Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO'S werden herstellt durch Kristallisation aus wäßriger Mischung bei 100 bis 250°C und autogenem Druck während 2 h bis 2 Wochen, wobei die Reaktionsmischung aus Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3 \times H_2O$ in 700 ml Wasser über 2 h zugetropft. Dabei wird der pH-Wert durch gleichzeitige Zugabe von 25%iger $NH_3$-Lösung bei pH 8 gehalten. Der entstandene Niederschlag wird 12 h nachgerührt, dann abgesaugt und ausgewaschen. Er wird bei 60°C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C, herstellen.

Auf diese Phosphate können auch Imprägnierung (Tränken und Aufsprühen) oder in manchen Fällen auch durch Ionenaustausch Modifizierungskomponenten, wie voran bei den Zeolithen beschrieben, aufgebracht werden. Auch kann wie bei den Zeolithkatalysatoren eine Modifizierung mit Säuren erfolgen.

Geeignete saure Katalysatoren sind z.B. auch die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Aluminiumoxide, Chromoxide, Molybdänoxide, Wolframoxide oder Bims oder

Gemische dieser Oxide. Auch können diese Oxide durch Aufbringung von Modifizierungskomponenten, wie voran bei den Zeolithkatalysatoren beschrieben, dotiert werden. Die Behandlung mit Säuren, wie voran bei den Zeolithkatalysatoren beschrieben, ist ebenfalls eine eventuelle Möglichkeit der Modifizierung.

Auch mit Phosphorsäure oder Borsäure getränkte Katalysatoren können eingesetzt werden. Phosphorsäure oder Borsäure wird z.B. auf $SiO_2$-, $Al_2O_3$-, $TiO_2$- oder Bimsträger, z.B. durch Auftränken oder Versprühen aufgebracht. Ein phosphorsäurehaltiger Katalysator kann beispielsweise durch Auftränken von $H_3PO_4$-, oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden ; danach schließt sich eine Trocknung und meist eine Calcination an. Phosphorsäure kann auch in einer Imprägniermühle auf das Trägermaterial aufgesprüht werden.

Die hier beschriebenen Heterogenkatalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung in Gegenwart eines Heterogenkatalysators in der Regel gewählten Reaktionsbedingungen sind in der Gasphase 100 bis 500, vorzugsweise 200 bis 400°C und eine Belastung WHSV = 0,1 bis 20 $h^{-1}$, bevorzugt 0,5 bis 5 $h^{-1}$ (g Edukt/g Katalysator und Stunde). Die Reaktion läßt sich in einem Festbett oder auch Wirbelbett ausführen.

Auch ist es möglich, die Reaktion in der Flüssigphase (Suspension-, Riesel- oder Sumpffahrweise) bei Temperaturen zwischen 50 und 200, insbesondere 80 bis 180°C, durchzuführen.

Das Verfahren wird in der Regel bei Normaldruck oder bei vermindertem oder erhöhtem Druck durchgeführt, wobei die Durchführung diskontinuierlich, aber vorzugsweise kontinuierlich erfolgen kann.

Das Edukt kann in gelöster Form z.B. in THF-, Toluol- oder Petrolether-Lösung zum Einsatz gebracht werden. Generell ist eine Verdünnung des Eduktes mit derartigen Lösungsmitteln oder mit Inertgasen wie $N_2$, Ar, $H_2O$-Dampf möglich. In besonderen Fällen kann auch $O_2$ verwendet werden.

Nach der Umsetzung können die entstandenen Produkte durch übliche Techniken z.B. durch Destillation aus dem Reaktionsgemisch isoliert werden. Nichtumgesetztes Einsatzgemisch wird gegebenenfalls für die erfindungsgemäße Umsetzung zurückgeführt.

Besonders vorteilhaft wird die Arbeitsweise dadurch, daß die gasförmigen Reaktionsprodukte sofort in eine Trennung eingebracht und anschließend in ihre Einzelkomponenten zerlegt werden. Eine derartige Trennung kann z.B. in einer Fraktionierkolonne durchgeführt werden.

Bei Verwendung homogener Katalysatoren wie Mineralsäuren, organischen Säuren oder Anhydriden wird die Dehydratisierung und Abspaltung der Schutzgruppe vorteilhaft in Abwesenheit eines Lösungsmittels bei Temperaturen von 80 bis 180, insbesondere 120 bis 140°C vorgenommen. Es ist auch möglich, die Umsetzung in Gegenwart hochsiedender Lösungsmittel wie Ethylenglykol, Phthalester, Silikonöl oder hochsiedender Mineralölfraktionen als Wärmeüberträger vorzunehmen.

In der Regel erfolgt die Umsetzung bei Normaldruck, ein geringer Unterdruck von ca. 100-300 mbar kann unter Umständen vorteilhaft sein.

Die Menge an homogenem Katalysator ist nicht besonders kritisch. Allgemein werden 0,01-5, insbesondere 0,1-4, besonders bevorzugt 1-2 Mol%, bezogen auf den Allylalkohol II, eingesetzt. Größere Mengen sind möglich, bringen aber keine weiteren Vorteile.

Die nachfolgenden Beispiele veranschaulichen die Erfindung.

Beispeil 1

Herstellung von 9-Hydroxydodec-10-enyl-1-tert.-butylether (4)

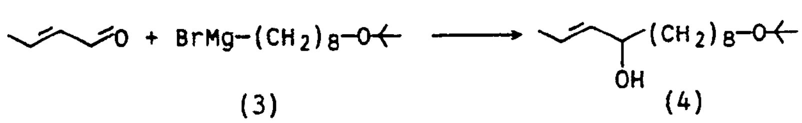

(3) (4)

434 g (1,64 mol) 8-Bromoctyl-t-butylether (3) und 50 g (1,9 mol) Magnesium wurden in 2 l THF (Tetrahydrofuran) in üblicher Weise mit 105 g (1,5 mol) Crotonaldehyd in 200 ml THF bei −10°C langsam versetzt. Man ließ 1 h bei −10°C nachrühren, hydrolysierte mit 2 l Eiswasser, säuerte bis pH 3 an und trennte die organische Phase ab. Die Wasserphase wurde mit Toluol mehrfach extrahiert und das Extrakt zusammen mit der Hauptmenge über $Na_2SO_4$ getrocknet, eingeengt und destilliert.

Ausbeute : 296 g der Verbindung (4) = 77% d. Th.

Sdp. 127°C/0,1 mbar

Beispiele 2 und 3

Herstellung von 8,10-Dodecadien-1-ol (5) unter Verwendung eines homogenen Katalysators

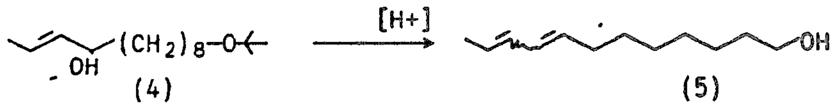

(4) (5)

533 g (2,08 mol) der Verbindung (4) wurden mit 5,5 g p-Toluolsulfonsäure versetzt und auf 140°C erwärmt. Dabei destilliert die stöchiometrische Menge Wasser ab. Weiteres Erwärmen auf 170°C erzeugt starke Gasentwicklung und Isobuten entweicht. Nach beendeter Gasentwicklung läßt man abkühlen und destilliert unter vermindertem Druck.

Ausbeute : 318 g $\hat{=}$ 84% (Isomerengemisch)

Sdp. 120°C/0,1 mbar

Das Isomerengemisch enthält ca. 50-60% E,E-8,10-Dodecadien-1-ol, das durch Kristallisation bei entsprechend tiefer Temperatur isoliert werden kann.

Entsprechend Beispiel 2 wurde die Umsetzung in Gegenwart von 1 Mol% konzentrierter $H_2SO_4$ anstelle von p-Toluolsulfonsäure durchgeführt.

Ausbeute 341 g $\hat{=}$ 90% (Isomerengemisch)

Beispiele 4-12

Herstellung von 8,10-Dodecadien-1-ol unter Verwendung acider Heterogenkatalysatoren

Die Umsetzung von 9-Hydroxydodec-10-enyl-1-tert.-butylether wurde in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

Die Abtrennung des 8,10-Dodecadien-1-ol-Isomerengemisches wird durch Destillation unter vermindertem Druck vorgenommen und die Isolierung des E,E-8,10-Dodecadien-1-ols geschieht durch Kristallisation bei entsprechend tiefer Temperatur.

Die für das erfindungsgemäße Verfahren eingesetzten Katalysatoren sind :

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 :50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% $SiO_2$ und 2,3 Gew.% $B_2O_3$.

Katalysator A wird erhalten, indem man den Borosilikatzeolith mit einem Verformungshilsmittel zu 2-mm-Strängen verformt, bei 110°C/16 h getrocknet und bei 500°C/24 h calciniert.

Katalysator B

Katalysator B wird hergestellt, indem man Katalysator A mit $Ce(NO_3)_2$ dotiert, bei 130°C/2 h trocknet und bei 540°C/2 h calciniert. Der Ce-Gehalt beträgt 1,8 Gew.%.

Katalysator C

Katalysator C wird hergestellt wie Katalysator C, indem man jedoch statt mit Ce-Nitrat mit $Cs_2CO_3$ dotiert. Der Cs-Gehalt beträgt 0,6 Gew.%.

Katalysator D

Siliciumaluminiumphosphat-5 (SAPO-5) wird aus einer Mischung aus 200 g 98%iger Phosphorsäure, 136

g Boehmit, 60 g Kieselsol (30%ige), 287 g Tripropylamin und 587 g $H_2O$ hergestellt. Diese Mischung wird bei 150°C während 1168 h unter autogenem Druck umgesetzt. Nach Filtration wird das kristalline Produkt bei 120°C getrocknet und bei 500°C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfmittel zu 3-mm-Strängen verformt, bei 120°C getrocknet und bei 500°C calciniert.

Katalysator E

Kommerziell erhältliches Zirkonphosphat $Zr_3(PO_4)_4$ in Reinsubstanz verformt.

Katalysator F

$BPO_4$ wird hergestellt, indem man 49 g $H_3BO_3$ mit 117 g $H_3PO_4$ (75%ig) in einem Kneter zusammengibt, überschüssiges Wasser abdampft und das Umsetzungsprodukt zu 3-mm-Strängen verformt. Diese Stränge werden bei 100°C getrocknet und bei 350°C calciniert. Katalysator D enthält 8,77 Gew.% B und 28,3 Gew.% P.

Katalysator G

200 g eines im Handel erhältlichen $SiO_2$ (D 11-10®) werden 1 h mit 600 ml 15%iger HCl bei 80°C behandelt. Danach wird das Material chloridfrei gewaschen, bei 110°C getrocknet und 1 h bei 600°C calciniert.

Die mit diesen Katalysatoren erzielten Versuchsergebnisse und Versuchsbedingungen sind in der nachfolgenden Tabelle zusammengefaßt.

Tabelle

| Beispiel | 4 | 5 | 6 | 7 | 8 | 9 | 10 | 11 |
|---|---|---|---|---|---|---|---|---|
| Katalysator | A | A | B | C | C | E | F | G |
| Temperatur [°C] | 300 | 350 | 300 | 300 | 300 | 300 | 300 | 350 |
| WHSV [$h^{-1}$] | 2,0 | 2,0 | 2,5 | 2,0 | 2,0 | 2,0 | 2,0 | 2,0 |
| Umsatz [%] | 100 | 100 | 100 | 100 | 100 | 100 | 100 | 100 |
| Selektivität 1) | 78,9 | 85,1 | 85,4 | 88,3 | 81,2 | 75,4 | 84,5 | 73,5 |
| Selektivität 2) | 40,4 | 36,9 | 37,7 | 45,2 | 38,3 | 37,9 | 43,7 | 39,7 |

1) Isomerengemisch von 8,10-Dodecadien-1-ol

2) E,E-8,10-Dodecadien-1-ol

## Ansprüche

1. Verfahren zur Herstellung von Dien-1-olen der allgemeinen Formel I

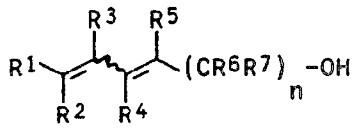

(I),

in der die Reste $R^1$ bis $R^7$ untereinander gleich oder verschieden sind und für Wasserstoff oder geradkettige oder verzweigte Alkylgruppen mit 1-12 Kohlenstoffatomen stehen und n = 1-14 bedeutet, dadurch gekennzeichnet, daß man Hydroxyalkenyl-tert.-butylether der Formel II

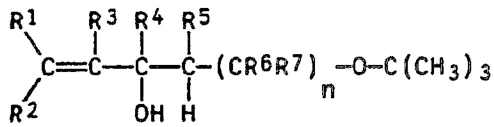

(II)

in Gegenwart von sauren Katalysatoren bei erhöhter Temperatur dehydratisiert und dabei im wesentlichen gleichzeitig die tert.-Butylschutzgruppe abspaltet.

2. Verfahren nach Anspruch 1, dadurch gekennzeichnet, daß man als Katalysator eine Mineralsäure, eine organische Säure oder Säureanhydride verwendet.

3. Verfahren nach den Ansprüchen 1 und 2, dadurch gekennzeichnet, daß man als Katalysator Schwefelsäure oder p-Toluolsulfonsäure verwendet.

4. Verfahren nach Anspruch 1, dadurch gekennzeichnet, daß man als Katalysator acide Zeolithe verwendet.

5. Verfahren nach den Ansprüchen 1 und 4, daß man als Katalysator Aluminium-, Boro- und/oder Eisensilikatzeolithe des Pentasiltyps verwendet.

6. Verfahren nach Anspruch 1, dadurch gekennzeichnet, daß man die Dehydratisierung und Abspaltung der Schutzgruppe in Gegenwart eines Heterogenkatalysators in der Gasphase bei Temperaturen von 100 bis 500°C vornimmt.

7. Verfahren nach Anspruch 1, dadurch gekennzeichnet, daß man die Dehydratisierung und Abspaltung der Schutzgruppe in Gegenwart eines Homogenkatalysators in der Flüssigphase bei Temperaturen von 80 bis 180°C vornimmt.

8. Verfahren nach Anspruch 1, dadurch gekennzeichnet, daß man 8,10-Dodecadien-1-ol Ia

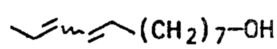

Ia,

ausgehend von 9-Hydroxy-dodec-10-enyl-1-tert.-butylether IIa

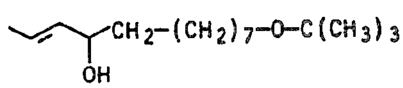

IIa

herstellt.

9. 9-Hydroxydodec-10-enyl-1-tert.-butylether.

10. Verfahren zur Herstellung von 8,10-Dodecadien-1-ol, bei dem man in an sich bekannter Weise aus 1,8-Octandiol den 8-Halooctyl-t-butylether herstellt, diesen in ebenfalls bekannter Weise mit Crotonaldehyd der Grignard-Umsetzung unterwirft, dadurch gekennzeichnet, daß man den erhaltenen 9-Hydroxydodec-10-enyl-1-tert.-butylether durch Erwärmen in Gegenwart eines sauren Katalysators im wesentlichen gleichzeitig dehydratisiert und die t-Butyl-Schutzgruppe daraus abspaltet.

## Claims

1. A process for preparing a dien-1-ol of the general formula I

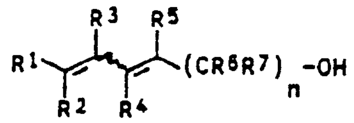

(I)

where the radicals $R^1$ to $R^7$ are identical to or different from one another and each is hydrogen or straight-chain or branched alkyl of 1-12 carbon atoms and n is 1-14, which comprises dehydrating a hydroxyalkenyl tert-butyl ether of the formula II

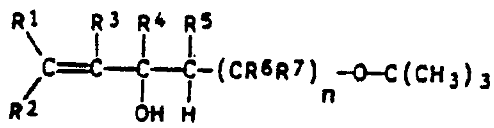

(II)

in the presence of an acidic catalyst at elevated temperatures and essentially at the same time eliminating the protective tert-butyl group.

2. A process as claimed in claim 1, wherein the catalyst used is a mineral acid, an organic acid or an acid anhydride.

3. A process as claimed in claim 1 or 2, wherein the catalyst used is sulfuric acid or p-toluenesulfonic acid.

4. A process as claimed in claim 1, wherein the catalyst used is an acidic zeolite.

5. A process as claimed in claim 1 or 4, wherein the catalyst used is an aluminosilicate, borosilicate or iron silicate zeolite of the pentasil type.

6. A process as claimed in claim 1, wherein the dehydration and elimination of the protective group is effected in the presence of a heterogeneous catalyst in the gas phase at from 100 to 500°C.

7. A process as claimed in claim 1, wherein the dehydration and elimination of the protective group is effected in the presence of a homogeneous catalyst in the liquid phase at from 80 to 180°C.

8. A process as claimed in claim 1, wherein 8,10-dodecadien-1-ol Ia

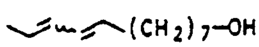

(Ia)

is prepared starting from 9-hydroxydodec-10-enyl 1-tert-butyl ether IIa

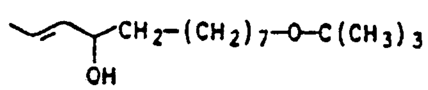

(IIa)

9. 9-Hydroxydodec-10-enyl 1-tert-butyl ether.

10. A process for preparing 8,10-dodecadien-1-ol, in which 1,8-octanediol is converted into an 8-halo-octyl t-butyl ether in a conventional manner and said ether is subjected to a Grignard reaction with crotonaldehyde, again in a conventional manner, which comprises the 9-hydroxy-dodec-10-enyl 1-tert-butyl ether obtained in the presence of an acidic catalyst to dehydrate said ether and at essentially the same time eliminate the protective t-butyl group therefrom.

## Revendications

1. Procédé de préparation de diène-1-ols de formule générale I

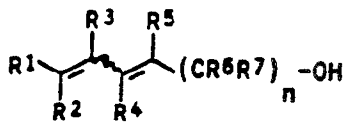

(I)

dans laquelle les symboles $R^1$ à $R^7$, ayant des significations identiques ou différentes, représentent chacun l'hydrogène ou un groupe alkyle en C 1-C 12 à chaîne droite ou ramifiée et n est un nombre allant de 1 à 14, caractérisé en ce que l'on soumet des oxydes de tert-butyle et d'hydroxyalcényle de formule II

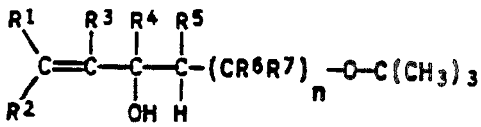

(II)

à déshydratation à haute température en présence de catalyseurs acides, avec séparation essentiellement simultanée du groupe protecteur tert-butyle.

2. Procédé selon la revendication 1, caractérisé en ce que l'on utilise en tant que catalyseur un acide minéral, un acide organique ou un anhydride d'acide.

3. Procédé selon les revendications 1 et 2, caractérisé en ce que l'on utilise en tant que catalyseur l'acide sulfurique ou l'acide p-toluène-sulfonique.

4. Procédé selon la revendication 1, caractérisé en ce que l'on utilise en tant que catalyseur une zéolithe acide.

5. Procédé selon les revendications 1 et 4, caractérisé en ce que l'on utilise en tant que catalyseurs des zéolithes du type pentasil consistant en silicates d'aluminium, borosilicates et/ou silicates de fer.

6. Procédé selon la revendication 1, caractérisé en ce que l'on procède à la déshydratation et à la scission du groupe protecteur en présence d'un catalyseur hétérogène en phase gazeuse, à des températures de 100 à 500 degrés C.

7. Procédé selon la revendication 1, caractérisé en ce que l'on procède à la déshydratation et à la scission du groupe protecteur en présence d'un catalyseur homogène en phase liquide, à des températures de 80 à 180 degrés C.

8. Procédé selon la revendication 1, caractérisé en ce que l'on prépare le 8,10-dodécadiène-1-ol de formule Ia

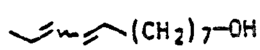

Ia,

au départ de l'oxyde de tert-butyle et de 9-hydroxydodéca-10-ényle-1 de formule IIa

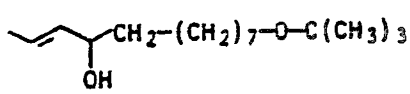

IIa

9. L'oxyde de tert-butyle et de 9-hydroxy-dodéca-10-ényle-1.

10. Procédé de préparation du 8,10-dodécadièn -1-ol dans lequel on prépare, de manière connue en soi, à partir du 1,8-octane-diol, un oxyde de tert-butyle et de 8-halogénooctyle et on soumet cet éther, de manière également connue en soi, à la réaction de Grignard avec l'aldéhyde crotonique, caractérisé en ce que l'on soumet l'oxyde de tert-butyle et de 9-hydroxy-dodéca-10-ényle-1 ainsi obtenu à déshydratation et scission essentiellement simultanée du groupe protecteur tert-butyle par chauffage en présence d'un catalyseur acide.